# EUROPEAN PATENT APPLICATION

(11) **EP 3 184 503 A1**
(43) Date of publication of application: **28.06.2017**
(21) Application number: 15833187.6
(22) Date of filing: 20.08.2015
(51) Int. Cl.: C07C 61/22, C12P 7/40, A61K 31/185, A61P 35/00, C07C 51/16

(54) **PERILLIC ACID DERIVATIVE, METHOD FOR PREPARING A PERILLIC ACID DERIVATIVE, PHARMACEUTICAL COMPOSITION, USE OF A PERILLIC ACID DERIVATIVE AND CANCER TREATMENT METHOD**

(30) Priority: 20.08.2014 BR 102014020443
(71) Applicant: Fundação Oswaldo Cruz, 21045-900 Rio de Janeiro, RJ (BR)
(72) Inventor: SIANI, Antonio Carlos, 22020-000 Rio de Janeiro - RJ (BR); FERRARA, Maria Antonieta, 20270-260 Rio de Janeiro - RJ (BR); DE LACERDA, Paulo Sérgio Bergo, 122041-010 Rio de Janeiro - RJ (BR); LÓPEZ, Raquel Elisa da Silva, 22240-006 Rio de Janeiro - RJ (BR); SAMPAIO, André Luiz Franco, 24130-082 Niterói - RJ (BR); TAPPIN, Marcelo Raul Romero, 22240-006 Rio de Janeiro - RJ (BR); BON, Elba Pinto da Silva, 22450-130 Rio de janeiro - RJ (BR)
(74) Representative: Grünecker Patent- und Rechtsanwälte PartG mbB
(86) International application number: PCT/BR2015/000127
(87) International publication number: WO 2016/026014

(57) **Abstract**

The present invention provides a compound of perillic acid in the form of a salt, having Formula II, exhibiting anti-cancer activity, where counterion M⁺ comprises alkaline or alkaline earth metals, such as Na⁺ (sodium), K⁺ (potassium), Ca⁺²/2 (calcium), etc.

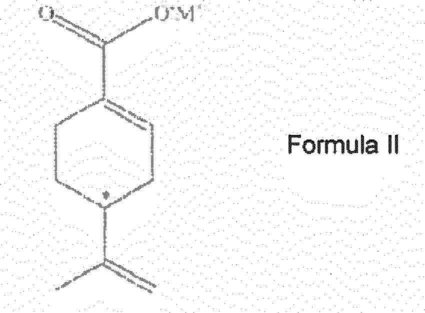

The invention further discloses a method of obtaining the compound, a pharmaceutical composition, use of the compound and a method of treating cancer.

## Description

### FIELD OF THE INVENTION

The research and development of anti-cancer drugs is a priority worldwide due to the exponential advance of the disease, however, researches are being carried out on the development of a drug that not only uses clean technology, but also low-cost raw materials, contributing to the reduction of expenses due to conventional chemotherapy. For example, we can mention the compounds derived from the citrus industry, in which Brazil occupies world leadership. One of these compounds is limonene, which is obtained from industrial waste, that is, citrus peels. Perillic acid (AP) (formula I) is known for its anti-cancer activity and can be produced from the action of microorganisms, especially yeasts belonging to the genera *Arxula*, *Candida* and *Yarrowia* on limonene, through biological synthesis (bioconversion).

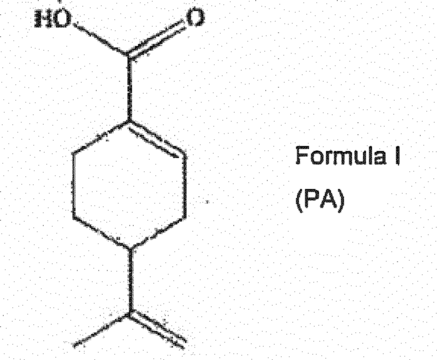

The present invention comprises compounds derived from perillic acid in the form of salt, having formula II, which can be obtained from perillic acid, which in turn, is obtained by the bioconversion of limonene, having anti-cancer activity and being soluble in water.

Compounds of salts deriving from perillic acid where the counter-ion M⁺ comprises alkaline or alkaline earth metals such as, for instance, Na⁺(sodium), K⁺ (potassium), Ca⁺² (calcium), which are represented by Formula II, where the counter-ion M⁺ can be Na, K, Ca/2, etc.

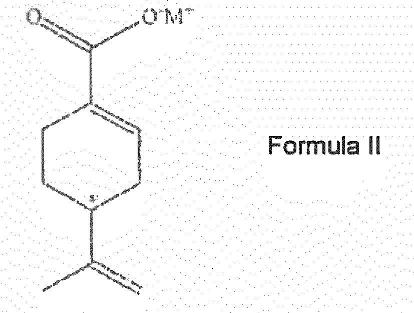

The present invention comprises, in particular the attainment of compounds deriving from perillic acid in the form of salt, such as sodium perylate, described herein as NAP (Formula III), having anti-cancer activity and being soluble in water.

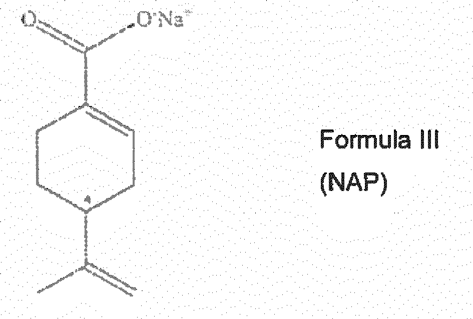

The present invention relates in particular to a compound, a pharmaceutical composition, process for obtaining a compound, use of a compound for treating cancer and method for treating cancer.

### BACKGROUND OF THE INVENTION

Limonene consists of a low-cost monoterpene, widely distributed in nature, being an abundant by-product of citrus industry and able to provide oxygenated derivatives that have high added value of great pharmaceutical importance. Among these derivatives, perillic acid occupies a prominent place because it has anti-cancer and anti-microbial activity, as well as perillyl alcohol and perilaldehyde, according to the structural formulas described below.

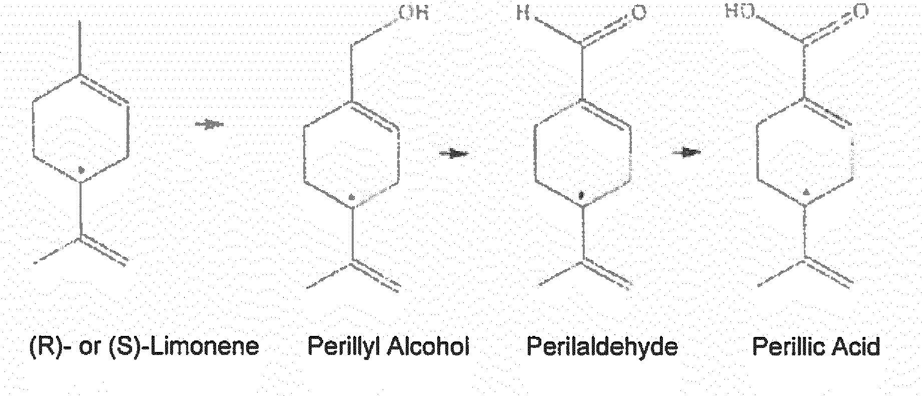

Bioconversion processes offer technical, economic and environmental advantages in comparison with chemical synthesis, among which we highlight the formation of products with chemo-, regio- and enantio-selectivity, with less formation of by-products, use of mild reaction conditions and low energy consumption. Industrial applicability of bioconversion has become increasingly important, especially to produce drugs and drug intermediates, due to the ability to produce enantiomerically pure products.

Due to the abundant availability of R-(+)-limonene dextrorotatory isomer from industrial citrus residues, the microbial bioconversion of this compound has been intensively exploited to establish low-cost methods to produce aromatic compounds (e.g., α-terpinol) and pharmacologically-active derivatives, such as perillyl alcohol and perillic acid, which are described in:
- Cadwallader K.R.; Braddock RJ.; Parish M.E.; Higgins D.P. Bioconversion of d-limonene by Pseudomonas gladioli. J. Food Sei. 54, 1241-1245 (1989);
- Mirata; MA.; Beerd D.; Schrader J. Integrated bioprocess for the oxidation of limonene to perillic actd with Pseudomonas putida DSM 12264, Process Biochem. 44, 764-771 (2009);
- Speetmans G.; Bijlsma A.; Eggink G. Limonene bioconversion to high concentrations of a single and stable product, perillic acid, by a solvent- resistant Pseudomonas putda strain. Appl. Microbiol. Biotechnol. 50, 538-544 (1998).

Derivatives obtained from limonene have been studied as anti-microbial and anti-cancer agents by the following authors:
- Da Fonseca, C.O.; Simao, M.; Lins, I. R.; Caetano, R. O.; Futuro, D.; Quirico-Santos, T. Efftcacy of monoterpeneperillyl alcohol upon survival rate of patients wrth recurrent glioblastoma J. Cancer Res. Clin. Oncot. 137. 287-293 (2011);
- Yeruva, L; Pierre, KJ.; Elegbede, A.; Wang, R.C.; Carper, S.W, Perillyl alcohol and perillic acid induced cell cycle and apoptosis in non-small cell lung cancer. Cancer letters 257, 216-226 (2007).

Patent application N°. WO95/24895 describes the use of perilyl alcohol and perillic acid methyl ester to reduce serum cholesterol levels to form and cause regression of the carcinoma; while patent document N°. **BRP10107262** refers to the treatment of cell tumors in the mammalian nervous system using perillyl alcohol to inhibit the formation and cause regression of tumors.

Perillic acid (PA) is commercially-available in the levorotatory form, or (S)-PA, such as perillyil alcohol: Thereby, it can be inferrred that (S)-AP is obtained by chemical synthesis involving several steps initiated with precursors other than (R)-limonene, since this would produce dextrorotary molecules.

Thus, the production of an anti-cancer drug obtained from a low-cost raw material is considered to be relevant to the population's health. This is represented by limonene, which is a derivative of the citrus industry, which is processed through a clean technology, without using solvents or toxic catalysts to produce the active principle. In contrast, the method uses an aqueous biological method to convert limonene by the action of a biologically safe yeast. Furthermore, the developed process makes use of only electric energy to stir the system, without the application of heat, that is, it is carried out at room temperature, with neutral pH (water pH). In this way, not only does the fermentation process takes advantage of the industrial waste, but it also generates biodegradable residues, thus meeting the principles of Green Chemistry.

It is important to emphasize that the subject matter of the present invention relates to a compound and pharmaceutical composition, whose production process has a high degree of sustainability.

### SUMMARY OF THE INVENTION

The present invention comprises a compound deriving from perillic acid in the salt form, according to Formula II, where counter-ion M⁺ comprises alkaline or earth alkaline metals such as, for instance, Na⁺ (sodium), K⁺ (potassium), Ca⁺² (calcium), etc. In particular, the compound of the invention is sodium perylate (NAP), represented by Formula III, obtained from perillic acid, which was produced by limonene bioconversion.

A further objective of the present invention is related to a pharmaceutical composition comprising compounds derived from perillic acid in the form of a salt of Formula II, in particular, the compound sodium perylate (NAP) of Formula III.

A further objective of the present invention is the attainment of compounds deriving from perillic acid in the form of salt of Formula II, in particular, the compound sodium perylate (NAP) of Formula III. Moreover, the invention relates to the use of compounds deriving from perillic acid in the form of a salt, in particular, sodium perylate (NAP), in treating cancer.

The present invention further provides a method of treating cancer using compounds derived from perillic acid in the form of a salt, in particular, sodium perylate (NAP).

### DESCRIPTION OF THE DRAWINGS

The content of each figure is briefly described below.
Figure 1 represents the flowchart of the process for obtaining a compound deriving from perillic acid.
Figure 2 shows the effect of (S)-NAP in HCT-116 (highly malignant human colon adenocarcinoma) viability. The linear regression shows a statistically significant correlation coefficient (R²) of 0.9347, using a 95% confidence interval.
Figure 3 shows the effect of (S)-NAP in HT-29 (human colon adenocarcinoma) tumor cell viability. The linear regression shows a statistically significant correlation coefficient (R²) of 0.9569, using a 95% confidence interval.
Figure 4 shows the result of the anti-proliferative assay on K562 (leukemia) cells.
Figure 5 shows the result of the anti-proliferative assay on MCF7 (breast tumor) cells.
Figure 6 shows the result of the anti-proliferative assay on Lucena (multi-drug resistant leukemia) cells.
Figure 7 shows the result of the anti-proliferative assay on SKMEL (melanoma) cells.

### DETAILED DESCRIPTION OF THE INVENTION

Perillic acid (AP) (Formula I) is known for its anti-cancer activity, and it can be produced by the biological synthesis (bioconversion) fostered by the action of microorganisms, in particular yeasts belonging to the genera *Arxula, Candida* and *Yarrowia*, on limonene. In turn, limonene is a compound classified as a monoterpene, obtained in large scale from the residues of the citrus industry (orange peels).

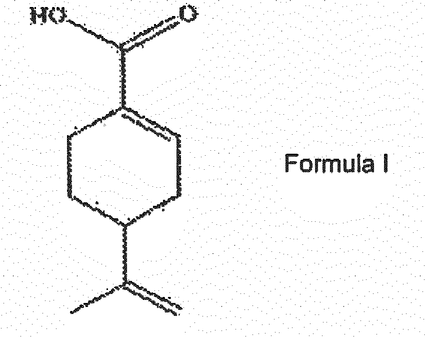

Based on the results of clinical and pre-clinical assays with perillyl alcohol (POH) and also with perillic acid (PA) described in the literature, compounds derived from perillic acid in the form of salt, in particular sodium perylate (NAP), have emerged as viable agents to prevent tumor growth. The main reasons to use compounds derived from perillic acid in the form of salt to treat tumors are the following:
(i) a high concentration of perilic acid and dihydroperillic acid was found in plasma from patients receiving clinical treatment with perillyl alcohol;
(ii) NAP solubility in an aqueous medium is higher than the one of POH and PA, and thus, it has a greater solubility in the physiological medium and blood plasma, which makes it more available in the body, leading to lower concentration requirement to achieve the same activity levels as the more lipophilic derivatives, such as perillic acid and perillyl alcohol;
(iii) the greater bioavailability of the compounds deriving from perillic acid salts allows the performance in the different organs of the body to be more comprehensive when compared with the more lipophilic molecules of perillyl alcohol or Perillic acid;
(iv) the acid functionality and the ionization potential of perillic acid salts facilitate the production of molecular hybrids or novel drug compositions, making possible combinations that also consolidate their use as an adjuvant and/or synergistic agent in conventional cancer treatments.

The present invention comprises the attainment of compounds deriving from perillic acid in the form of salt (Formula II), in particular sodium perylate, described herein as NAP (Formula III), having anti-cancer activity and being soluble in water. This molecule is obtained from perillic acid that, in turn, derives from limonene bioconversion.

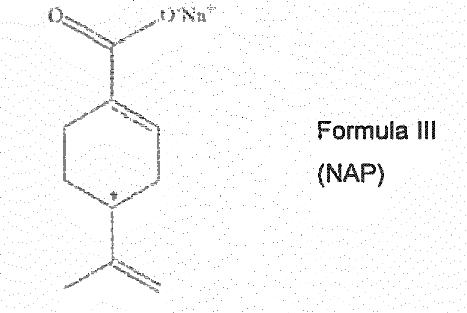

### PROCESS FOR OBTAINING PERILLIC ACID AND SODIUM PERYLATE

Before obtaining the compound of the present invention, sodium perylate (NAP), perillic acid must be ontained by means of limonene bioconversion.

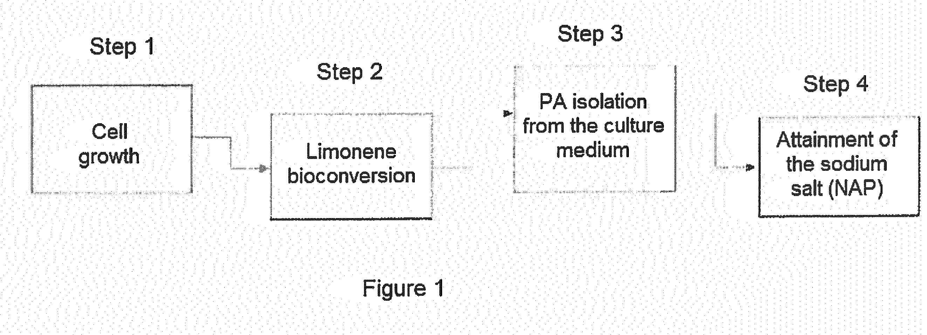

Through a biological process, the monoterpene limonene is converted into perillic acid by the action of microorganisms, in particular yeasts belonging to the genera *Arxula, Candida* e *Yarrowia*. The product of this bioconversion, perillic acid (AP), is extracted from the biological aqueous medium and, then, alone transformed into the corresponding salt, exemplified by the sodium salt NAP, resulting from the action of the appropriate base in a compatible solvent. The attainment process takes place in four main steps, as represented in the flowchart of Figure 1.

The process for obtaining sodium salt (NAP), for instance, involves the following steps:
1) Cell growth
2) Limonene bioconversion
3) Isolation of perillic acid from the culture medium
4) Attainment of sodium salt (NAP)

### Step 1 - Cell growth

At this stage, cell propagation is carried out from stock culture and biomass production.

The cell growth stage uses the selected yeast, having been classified as safe, according to GRAS (Generally Regarded as Safe) criteria, as established by the US Food and Drug Administration (FDA), body responsible for the registration of medicaments in the United States. At this stage, the ideal cell growth conditions, to produce enough biomass to start bioconversion were optimized by studies that considered parameters affecting cell culture, such as carbon source and nitrogen source.

### Step 2 - Limonene bioconversion

According to the prior art, we found that *Yarrowia lipolytica* is a microorganism producing specific enzymes, that are capable of oxidate limonene into perillic acid. Limonene bioconversion to obtain perillic acid (PA) takes place in one single step, which was tested to produce maximum yield, with the following parameters: a) concentration of yeast cell mass between 5-20 g/L; b) amount of limonene used in the reaction between 0.1% and 0.5% v/v; c) addition of limonene in a single portion or in intermittent portion; d) pH of the medium ranging between 5.7-7.4; and) temperature ranging from 20 to 30 °C; and, f) reaction time from 24 to 48 h.

The obtained amount of PA is comparable with what is reported for bioconverters similar to limonene, using bacteria, that is, about 1g/L of limonene, corresponding to a 30% conversion, when performed at the same scale.

One of the advantages of the process of obtaining perillic acid over the prior art comprises the selective production of the PA by bioconversion, that is, the resulting by-products do not contaminate the product such as perylaldehyde, perillyl alcohol, and substances derived from polyhydroxylated limonene.

Furthermore, bioconversion involves an aqueous medium, thus eliminating all the toxic components, complying with the current environmental requirements.

### Step 3 - Extraction of the perillic acid from the culture medium

In this step, AP is precipitated in acid medium or, alternatively, extracted with an appropriate solvent.

Thus, after completion of the limonene bioconversion step, the third step of the process, involving the step of isolating perillic acid (PA), which is stable enough under normal ambient conditions, is started. Given its monoterpene nature (having 10 carbons) and the presence of a carboxylic function, the PA can be isolated in several ways, among which two were chosen:
(1) partitioning with the appropriate solvent: the PA isolation can be performed by liquid-liquid partitioning, using a water-immiscible lipophilic solvent, particularly ethyl acetate; or,
(2) combination of filtration with acid-base partition: the extraction can be effected by an idealized set of operations that include, in some of its stages, the increase/decrease of the pH of the medium, with mineral acids and alkali metal hydroxides, pre- or post-filtration on suitable membranes; followed by the recovery of the final acid medium causing precipitation of the AP, which is then finally separated by filtration.

At this stage, the PA was isolated for two purposes: the former of them is for later attainment of the NAP or other salt derived from perillic acid; and the latter is for the attainment of samples that will be quantitatively analyzed, in order to evaluate the efficiency or not of limonene biooxidation by the yeast and the quality/purity of the obtained PA.

### Step 4 - Attainment of sodium salt (NAP)

Upon isolation of the perylic acid (PA), the fourth and final step of the process involves the attainment of perylate salt by replacing the hydrogen of the carboxylic acid with cations from alkali or alkali earth metals, such as Na⁺ (sodium), K⁺ (potassium), Ca⁺² (calcium), etc. This step aims at increasing the compound solubility in hydrophilic solvents to favor the solvation, facilitating the stabilization of the separation between the anions and the corresponding cations. Therefore, the change of covalent molecules into salts aims at facilitating the pharmacotechnical procedures as well as the biological availability of the drugs.

As for perillic acid (PA), it was found the existence of two enantiomeric forms, which can be obtained in the following manners:
- (S)-PA (levorotatory form)- purchased in the reagent market; or
- (R)-PA (dextrorotatory form)- produced in laboratory, by the conversion of (R)-limonene.

Both forms (levorotatory and dextrorotatory) can produce the corresponding salts, without prejudice to the optical activity (enantiomerism) inherent to each form, as long as the change of perillic acid (PA) into its corresponding salt does not affect its properties. Thus, independently of using one or the other enantiomer (R or S) of perillic acid (PA), the description of its change in perillic acid salt such as, for instance, NAP, does not need to specify this condition, because the chiral center of the molecule, responsible for this activity, will not be altered by transformation of this sort. Hence, the original PA enantiomer will be integrally maintained, that is, (R)-PA will generate only (R)-NAP, and (S)-PA will generate only (S)-NAP.

The transformation of PA in the NAP salt was carried out both for the commercial (S)-PA reagent, and for the (R)-PA reagent produced in the lab by bioconversion of (R)-limonene with *Y. lypofilica*.

In this case, both salts (R)-NAP and (S)-NAP were obtained by the action of an alkaline metal methoxide, in particular sodium methoxide commercially purchased, solubilized in the corresponding alcohol, in particular methanol. PA is relatively soluble and NAP (due to its ionic characteristics) is relative insoluble in alcohols. Hence, the controlled addition of a solution of sodium methoxide in methanol (10%-30%) to another methanolic solution of PA in suitable concentrations under stirring and ice-bath cooling, promotes the formation of a precipitate as shown in Example 5.

The formation of the NAP precipitate in methanol and the maximum PA consumption is ensured using a slight stoichiometric excess of sodium methoxide. This precipitate comprises a white to slightly yellowish-colored solid and can be filtered, washed with acetone, purified, dried and stored for antitumoral activity testing.

To obtain NAP two different routes can be used, based on: (1) the reaction of commercial (S)-PA with sodium methoxide, and (2) the biological synthesis of (R)-PA by (R)-limonene bioconversion with *Yarrowia lipolytica*, followed by the reaction of the latter with sodium methoxide to produce (R)-NAP, as described in the following scheme:

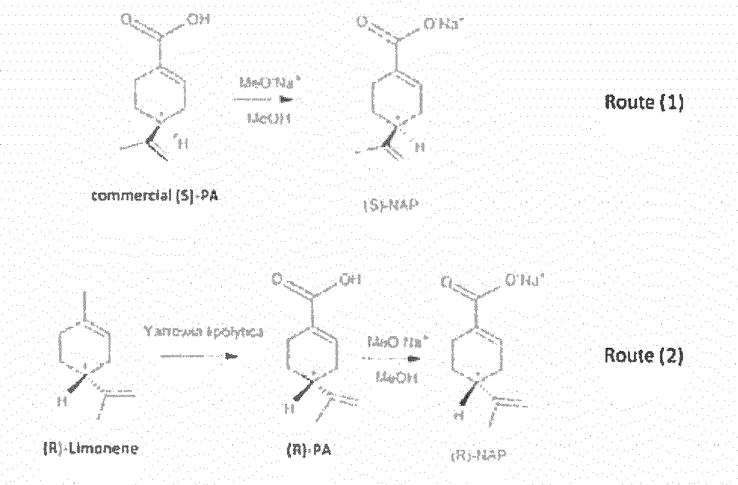

NAP was identified by conventional spectrometry, in particular by Proton Magnetic Resonance (¹H-NMR). This identification was supported by comparative data available for perillic acid, as well for limonene.

The yields of (S)-NAP and (R)-NAP varies from 44% to 76%, as measured by gas chromatography (GC); also used to determine the respective purity degrees. There are no citations describing this compound or data associated thereto; which makes it novel.

For example, date of ¹H-NMR obtained for R-NAP are listed below, according to Formula IV. The values indicated for compound R-NAP are identical to the ones of S-NAP, since the applied NMR technique does not differentiate enantiomers.

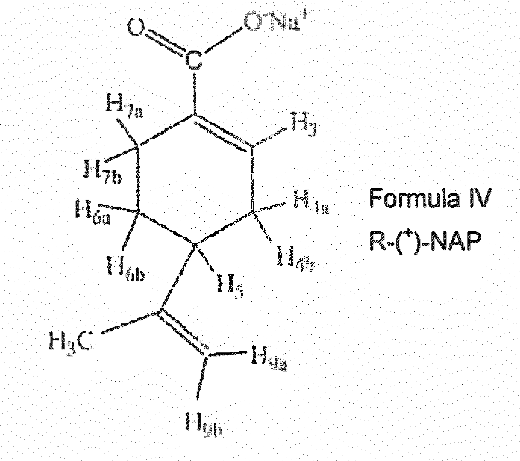

| **Proton** | **Indication (multiplicity)** |
|---|---|
| H-3 | 6.62 ppm (m) |
| H-4a | 2.27 - 2.29 ppm (m) |
| H-4b | 2.04 - 2.11 ppm (m) |
| H-5 | 2.16 - 2.22 ppm (m) |
| H-6a | 1.83 - 1.88 ppm (m) |
| H-6b | 1.41 - 1.50 ppm (dq) |
| H-7a | 2.16 - 2.22 ppm (m) |
| H-7b | 2.27 - 2.29 ppm (m) |
| H-9a | 4.81 ppm (s) |
| H-9b | 4.78 ppm (s) |
| H-10 (CH3) | 1.76 ppm (s) |

Examples are described below to provide details of the invention.

### Example 1

### Attainment of cell biomass of Yarrowia lipolytica

The used yeast lineage, *Y. lipolytica* ATCC 18942, used in limonene bioconversion, is based on the collection of foreign cultures (American Type Culture Collection), there being no characterization of access to resources of the genetic heritage of the Brazilian territory. The origin of the used strain, *Y*. *lipolytica,* is the institutional collection of microorganisms of the Fundaça̅o Oswaldo Cruz, whose deposit is stored in the Instituto Nacional de Controle da Qualidade em Saúde - Brazilian National Institute of Health Quality (INCQS/FIOCRUZ), under record N°. 40149.

The best yeast culture condition involved its growth in YMB (Yeast Malt Broth) (10 g/L glucose, 3.0 g/L yeast extract, 3.0 g/L malt extract, and 5.0 g/L peptone) on a rotary stirrer (250 rpm) at 25 °C for 48 h. The culture was carried out in 250ml Erlenmeyer flasks containing 50 ml of culture medium and the cells were incubated at 28 °C, under stirring at 250 rpm for48 h in a rotative stirrer. Cell mass concentration was determined at OD₆₀₀, with reference to a calibration curve. A unit in OD₆₀₀ corresponds to a dry cell concentration of 0.444 g/L of *Y*. *lipolytica*.

### Example 2

### Limonene bioconversion using Y. lipolytica

The cell mass of *Y*. *lipolytica* yeast, obtained as described in Example 1, was centrifuged at 3000 g and used for R-(+)-limonene conversion (96%- Fluka, Buchs, Switzerland). The reaction conditions of limonene bioconversion were the following ones: (i) temperature between 20 and 30 °C; (ii) pH between 5.7 and 7.4 in phosphate buffer (50 mM); (iii) cell mass concentrate between 5 g/L and 20 g/L; and (iv) limonene concentrate from 0.1 to 0,5 % (v/v). The reaction mixture was incubated under stirring at 250 rpm, and the cells were separated from the supernatant (containing PA) by centrifugation at 3000 g. According to the GC-FID analysis (see below), limonene bioconversion resulted in the formation of 1 g/L of perillic acid.

The production of perillic acid in the bioconversion was measured by gas chromatography with flame ionization detector (GC-FID). To this end, the samples were analyzed in two different preparations, as described in Examples 3A and 3B.

The GC analysis were performed in a gas chromatographer (model Network HP 6890N) with a capillary column HP-Innowax (30m x 0.25 mm x 0.25 mm of film thickness), a flow rate of 2.0 mL/min and a flow division rate mode 1/15 (injector "split/splitless"). The temperature program was as follows: initial temperature of 50°C, kept for 2 minutes, final temperature of 250 °C, kept for 8 minutes, and a heating rate of 10°C min. Helium was used as transport gas. The injector temperature was adjusted to 270 °C. Detection was carried out with a flame ionization detector (FID), at 270 °C. To detect the formed PA, a calibration curve was built using commercial (S)-perillic acid.

The structure of the perillic acid obtained through bioconversion was confirmed by gas chromatography coupled with mass spectrometry by electron impact (GC-EI). The analyses were carried out in the same conditions used in the GC-FID, except for the use of a HP-5 MS column. In this column, perillic acid is eluted in 13.03 minutes, resulting in a mass fragmentation identical to the commercial compound [m/z 166 (M⁺), 121 (⁺- CO₂H), 105, 93, 79, 68].

### Example 3

### Isolation of perillic acid from the reaction medium

### Example 3A: Extraction by solvent

The aqueous phase coming from limonene bioconversion was transferred to an extractor of 1 L, and NaCl (75 g/L) was added, stirring until complete soubilization of the salt. The aqueous phase was extracted with ethyl acetate (three portions of 325 mL/L) and separated from the organic phase in a separating funnel. After the organic phases were combined, the drying agent Na₂SO₄ (10 to 15 g/L) was added. Solvent filtration and concentration in a rotary evaporator led to the attainment of a slightly yellowish solid, which was then dried under high vacuum. The average yield of the process ranged between 80 and 90%, and the average purity of the sample ranged between 80 and 85%, as determined by GC. Once performed on a smaller scale, this procedure was also used to prepare samples for analysis by GC of the obtained PA.

### Example 3B: PA precipitation by acid addition

An aliquot (1 ml) of the aqueous phase coming from the limonene was transferred to a centrifugation tube, to which 50 µL of HCl 0.6 M were added to induce precipitation. Upon centrifugation at 10,000 rpm for 10 minutes and supernatant discard, the precipitate was dissolved into 2 ml of ethyl acetate, for GC analysis. The sample preparation method was evaluated using an artificial preparation of commercial perillic acid, obtaining a 98.1% recovery. Once carried out on a larger scale, this procedure was also used to extract PA from the bioconversion medium.

### Example 4

### Sodium perylate (NAP) preparation

In a solution of perillic acid (580-702 mg; 3.5-4.2 mmol) in MeOH (5.3-7.3 mL) a commercial solution of MeONa (30%, Vetec) was added with cooling (10-0°C) until complete precipitation. The filtrate was filtered and purified by washing with ice-cold acetone (8.5 to 10.5 mL) providing, after vacuum drying, a slightly yellowish solid corresponding to NAP (480 to 722 mg, yield from 73 to 91%). This process was performed for both commercial reagent (S)-PA, and for (R)-PA produced in the laboratory by (R)-limonene bioconversion; obtaining (S)-NAP and (R)-NAP, respectively. The obtained product was identified by conventional spectrometry, in particular ¹H-NMR.

### Example 5

### Tumoricidal effect of (S) -NAP molecule on highly malignant human colon adenocarcinoma cells - HCT-116

This example was carried out to prove the tumoricidal capacity of sodium perylate. The effect of (S)-NAP on the viability of human colon adenocarcinoma cells with increasing degrees of malignancy was first evaluated. Caco2, HT-29, HCT-116 and a normal control cell MDCK, a Dalmatian kidney cell. Proliferation assays
(i) Cell cultures: Lineages of human colon adenocarcinoma cells with decreasing malignancy levels, HCT 116, HT-29 and Caco-2, were used. As a control of the epithelial cell model, a normal cell line of drainage epithelium (IEC-6) was used. These cells were cultured according to the ATCC specifications and maintained in a DMEM medium in an oven, with 5% CO₂, at 37 °C until reaching semiconfluence.
(ii) Treatments: Initially, concentrations of sodium perylate (1.0, 1.8, 2.6, 3.4, 4.2 and 5.0 mM) were tested to determine the IC₅₀ of the drug. Monolayers of semiconfluent cells were serum-depleted for 24 hours before treatment with (S)-NAP and during the treatment for 24h.
(iii) Viability test: The lineages were plated in 96-well flat-bottom plates with DMEM culture medium containing the different (S)-NAP concentrations described above. Cell incubation with (S)-NAP was conducted for 24 h, at 37 °C, upon which, the plates were incubated for 2 h with MTT [3-(4,5-dimethylthiazol-2- yl)-2,5diphenyltetrazolium bromide] 2.5 mg/ml, at a final concentration of 250 µg/ml. The plates were centrifuged at 1200 RPM, at 4°C, for 5 minutes, poured to remove the supernatant, since the crystals had deposited inside the cells. Formazan crystals were dissolved into 100 µl of DMSO and homogenized manually and through the device. Absorbances were determined at 538 nm in a plate reader (Spectra Max 190). Controls of 100% viability, with no drug addition, and of 0% viability using formaldehyde were carried out simultaneously with the experiment.

### Results

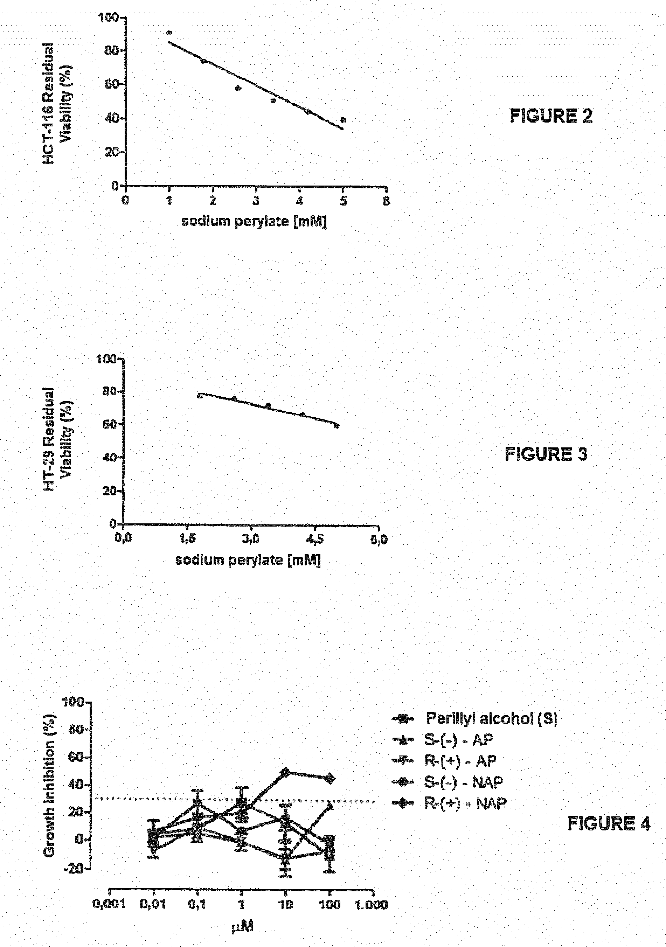

CaCO₂ cells proved to be refractory to treatment and the most aggressive cell, HCT-116, was the most affected one. Therefore, HCT-116 was used to proceed with the tests and CaCO₂ as negative control. The tumoricidal effect of (S)-PA is shown in Figures 2 and 3 and in Table 1.

**Table 1 - in vitro assay with (S)-NAP**

| **Lineage *(in vitro*)** | **NAP Dosage mM** | **Target** | **Result** |
|---|---|---|---|
| human colon adenocarcinoma (HT-29) | 1.5-5.0 | Colon tumor cells | IC₅₀ > 5.0 mM* |
| human colon | 1.0-5.0 | Colon tumor cells | IC₅₀ > 3.4 mM |
| adenocarcinoma (HCT-116) | | | |
| human colon adenocarcinoma (Caco-2) | 1.5-5.0 | Colon tumor cells | Refractory |
| Rat intestinal epithelial cell (IEC-6) | 1.5-5.0 | Cell model used as experiment control | - |

| | | | |
|---|---|---|---|
| Concentration limited by NAP solubility above this value. | | | |

### Example 6- Antiproliferative effect using (R)-AP, (S)-AP, (R)-NAP and (S)-NAP

Example 6 was carried out to determine the biological activity of the following samples: (S)-perillyl alcohol, (S)-AP, (R)-AP, (S)-NAP, and (R)-NAP on human tumor lineages. (S)-perillyl alcohol was tested in combination with the samples of sodium acids and salts, because it is a substance which is considered to have some established clinical efficacy.

### Proliferation Assay

Samples (R)-AP, (S)-AP, (R)-NAP, (S)-NAP and perillyl alcohol (POH) were received and solubilized in DMSO or saline solution (NaCl 0.9%) and conserved at -20 °C until the day of the experiment, when they were immediately diluted before use, in a cell culture medium, DMEM, enriched with 10% fetal bovine serum, 100 U/I penicillin, and 0.1 mg/ml streptomycin, and 0.05 mg/ml gentamicin (DMEM complete medium). The experiments were carried out according to the Standard Operational Procedure of the Laboratory of Molecular Pharmacology FARMO1003 version 00, which are summarized below.

K562 (leukemia), LUCENA (multi-drug resistant leukemia), SKMEL28 (melanoma) and MCF7 (breast tumor) cells were cultivated on a DEM complete culture medium, and kept in log growth phase. Twenty-four hours before the treatment with the samples, 100 µl of the cell suspension (5x10⁴ cells/ml) were added to wells of 96-well plates and kept in the oven at 5% CO₂ atmosphere, at 37°C.

The treatment for each of the five samples was carried out in response dosage (0.01 to 100 µM; 5 spots), each concentration in triplicate.

After 48 hours, thiazolyl blue was added (MTT; SIGMA code M5655) and the plate was kept in the oven with CO₂ for 4 hours. After the incubation time, the supernatant was aspirated and formazan crystals, formed by the cellular activity, were solubilized with isopropyl alcohol and, after full solubilization, the optical density determination was carried out in a microplate reader at 570 nm (VictorX5; Perkin Élmer USA).

### Results:

Figure 4 shows that the treatment with sample R-NAP (R-form sodium perylate) exhibited the best performance, with an IC₅₀ of 1.24 µM. The other tested samples did not show inhibition requiring the calculation of IC₅₀.

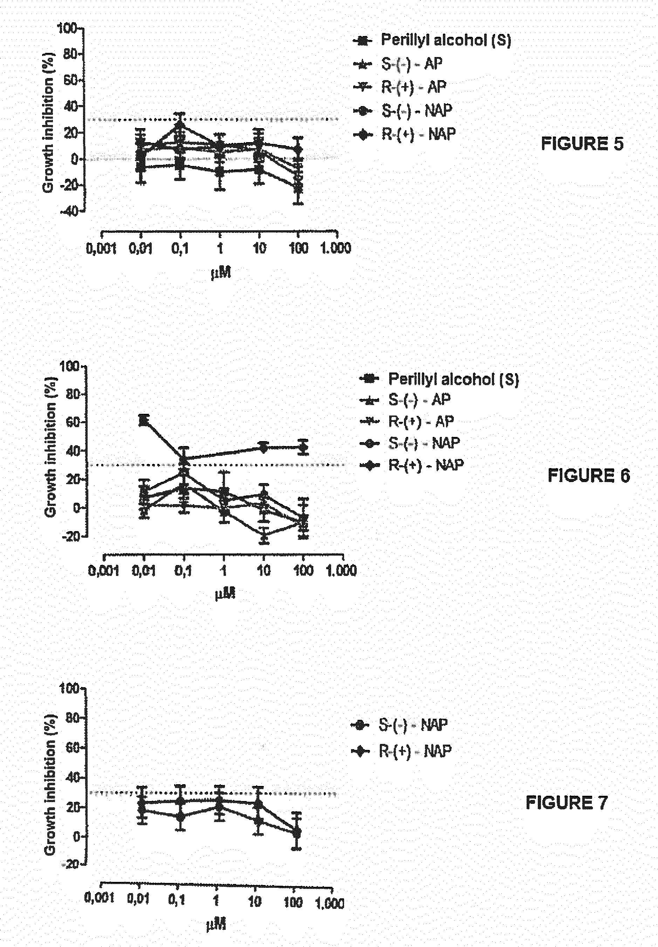

Figure 5 shows that the treatment with the five samples (R)-PA, (S)-PA, (R)-NAP, (S)-NAP and (POH) exhibit no inhibition making IC₅₀ calculation necessary.

Figure 6 shows that the treatment with sample (R)-NAP (R-form sodium perylate) exhibited the best performance, with an IC₅₀ of 0.013 µM. The other tested samples did not show inhibition requiring the calculation of IC₅₀.within the used dosage interval.

Figure 7 shows that treatment with samples (S)-PA (S-form sodium perylate) and (R)-NAP (R-form sodium perylate) exhibited no inhibition making IC₅₀ calculation necessary, within the used dosage interval.

Among the tested samples, the sample (R)-NAP (R-form sodium perylate) exhibit the best performance, showing activity on the K562 and LUCENA lineages, having a IC₅₀ of 1.240 and 0.013 µM, respectively, while the other tested samples showed no relevant activity on any of the lineages tested within the used dosage interval.

Based on the former results, it has been proven that the sodium salt of perillic acid, i.e., sodium perylate, is a product applicable to treat cancer, and it can also be used as synergistic adjuvant of conventional treatments.

The pharmaceutical composition of the present invention comprises sodium perylate obtained from perillic acid, the latter produced in the bioconversion process of limonene using *Yarrowia lipolytica* yeast as a single ingredient or in combination with an anti-cancer agent, said composition being formulated into a pharmaceutically-acceptable carrier, adjuvant and/or excipient suitable for use in humans, according to the conventional techniques for the preparation of pharmaceutical compositions.

Pharmaceutically acceptable carriers that may be used in the present composition comprise conventional pharmaceutical carriers, such as a phosphate buffered saline solution, water, and emulsions, such as water-in-oil emulsions or oil-in-water emulsions. The compositions may additionally contain solid pharmaceutical excipients such as starch, cellulose, talc, glucose, lactose, sucrose, gelatin, malt, rice, flour, chalk, silica gel, magnesium stearate, sodium stearate, glycerol monostearate, sodium chloride, powdered skimmed milk and similar substances.

Liquid and semi-solid excipients may be selected from glycerol, propylene glycol, water, ethanol, and various oils, including petroleum, animal, plant oils or oils of synthetic origin, for instance, peanut oil, soybean oil, mineral oil, sesame oil, *inter alia.* Liquid carriers, in particular for injectable solutions, include water, saline, aqueous dextrose, and glycols. As examples of suitable pharmaceutically-acceptable carrier, adjuvant and/or excipient, see Remington Pharmaceutical Sciences, edited by E.W. Martin (Mack Publishing Company, 18th ed., 1, 90). The compositions can also include stabilizers and preservatives.

As for the term "therapeutically effective amount" in the claims, it is to be understood that the therapeutically effective amount is an amount sufficient to treat a specific disorder or disease or, alternatively, to obtain a pharmacological response to the treatment of a disorder or disease. Methods of determining the most effective means and dosage of administration may vary with the composition used for the therapy, the purpose of the therapy, the target cell to be treated, and the patient to be treated. Treatment dosages can usually be titrated to optimize safety and efficacy. Single or multiple administrations may be performed at the dosage level and standard selected by the physician. Appropriate dosage formulations and methods of administering the agents may be readily determined by those skilled in the art. For instance, a composition is administered at about 0.01 mg/kg to about 200 mg/kg, at about 0.1 mg/kg to about 100 mg/kg, or at about 0.5 mg/kg to about 50 mg/kg. When the compounds described herein are co-administered with another agent or therapy, the effective amount may be less than when the agent is used alone. That is, said composition may be used in combination with another compound which also acts as an anti-cancer agent.

The pharmaceutical composition of the present invention comprises a compound derived from perillic acid in the form of a salt, according to Formula II in a therapeutically effective amount and a pharmaceutically acceptable carrier, adjuvant and/or excipient suitable for use in humans.

According to the present invention the compounds derived from perillic acid in the form of a salt, according to Formula II, where counter-ion M⁺ comprises alkaline or alkaline earth metals, such as Na⁺ (sodium), K⁺ (potassium), Ca⁺² (calcium), etc., and in particular sodium perylate (NAP), represented by Formula III, are used in manufacturing a medicament for use in the treatment, prevention or inhibition of carcinoma formation. The compound of Formula II may also be used in the treatment of diseases related to non-solid tumors such as leukemia, multiple myeloma, hematological malignancies or lymphoma, and to solid tumors and their metastases such as melanoma, non-small cell lung cancer, glioma, hepatocellular carcinoma, glioblastoma, thyroid carcinoma, bile duct, bone, gastric, cerebral/CNS, head and neck, hepatic, stomach, prostatic, breast, renal, testicular, ovarian, skin, cervical, pulmonary, uterine, vulvar, endometrial, colorectal, pancreatic, pleural/peritoneal, membrane, salivary gland and epidermoid tumors.

According to the present invention the compound derived from perillic acid in the form of salt of Formula (II) may be used to manufacture a medicament used in the production of an anti-cancer effect in a warm-blooded animal such as human being.

According to the present invention, the pharmaceutical composition comprising the compounds derived from perillic acid in the form of a salt, according to Formula II, where counter-ion M⁺ comprises alkaline or earth alkaline metals, such as Na⁺ (sodium), K⁺ (potassium, Ca⁺² (calcium, etc. and, in particular sodium perylate (NAP), represented by formula III, is used to manufacture a medicament to be used in carcinoma treatment, prevention and formation inhibition.

The invention further comprises methods for treating cancer comprising the compound of Formula (II), in particular the compound of Formula (III) (sodium perylate) as single ingredient or in combination with an anti-cancer agent.

## Claims

1. A compound **characterized in that** it has formula (II), 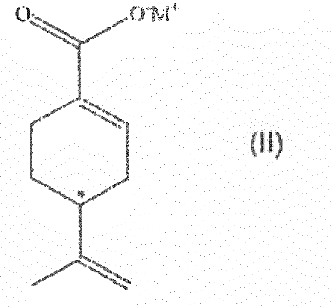 where counter-ion M⁺ comprises alkaline or earth alkaline metals.

2. The compound, according to claim 1, **characterized in that** counter-ion M⁺ is Na⁺ (sodium), K⁺ (potassium) or Ca⁺²/2 (calcium).

3. The compound, according to claim 1 or 2, **characterized in that** the compound comprises sodium perilate (NAP)of formula III.

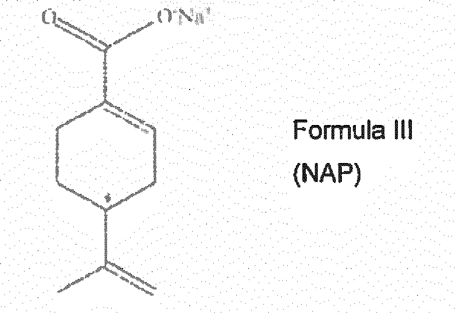

4. The compound, according to any one of claims 1-3, **characterized in that** the compound is obtained by the reaction of perillic acid and sodium methoxide (MeONa), wherein the solvent is methanol.

5. The compound, according to claim 4, **characterized in that** perillic acid is obtained by a bioconversion process of limonene, using *Yarrowia lipolytica* yeast.

6. A process for obtaining a compound, as defined in claims 1-5, **characterized in that** the perylate salt is obtained through a single step, according to route 1, wherein an alkali metal methoxide compound, in particular, sodium methoxide, solubilized in the corresponding alcohol, in particularly methanol, is added to the commercially purchased perillic acid.

7. The process for obtaining a compound, according to claim 6, **characterized in that** the sodium methoxide solution in methanol solution (10%-30%) is added to a methanol solution of perillic acid (AP), the addition being carried out in a controlled manner, at suitable concentrations, under stirring and ice bath-cooling.

8. The process for obtaining a compound, according to claim 6 or 7, **characterized in that** the compound comprises sodium perylate (NAP) of formula III.

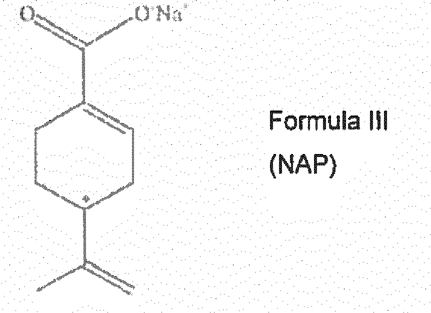

9. A process for obtaining a compound, as defined in claims 1-5, wherein said compound is obtained in four steps, according to route 2, consisting of: step 1, which involves cell culture growth and production of enough biomass to initiate bioconversion; step 2, which comprises limonene bioconversion using a yeast to produce specific oxigenated derivatives, *inter alia,* perillyl alcohol and/or perillic acid; step 3, which consists of isolating perillic acid (PA), by precipitation in the culture medium, through the addition of mineral acids, followed by separation and purification of the solid; or through the extraction by lipophilic water-immiscible solvent, or through the combination of the filtrate with acid-base partitioning upon perillic acid (PA) isolation; **characterized in that** step 4 comprises the attainment of perylate salt, by substituting the hydrogen of the carboxylic acid with a cation from alkaline or earth alkaline metals through the reaction of an alkaline metal metoxide, in particular sodium methoxide, solubilized in alcohol, in particular methanol.

10. The process for obtaining a compound, according to claim 9, **characterized in that** the sodium methoxide solution in methanol solution (10%-30%) is added to a methanol solution of perillic acid (PA), the addition being carried out in a controlled manner, at suitable concentrations, under stirring and ice bath-cooling.

11. The process for obtaining a compound, according to claims 9 or 10, **characterized in that** the obtained compound comprises sodium perylate (NAP) of Formula III.

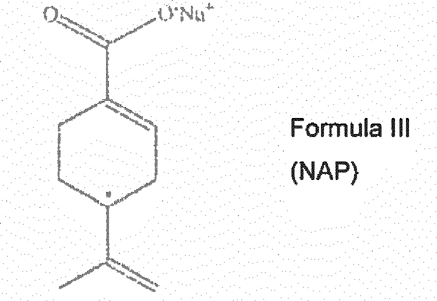

12. A pharmaceutical composition **characterized in that** it comprises a compound, as defined in any one of claims 1-5, in a therapeutically effective amount, and a pharmaceutically acceptable carrier, adjuvant and/or excipient suitable for use in humans.

13. The pharmaceutical composition, according to claim 12, **characterized in that** said composition is used in combination with another compound that also acts as an anti-cancer agent.

14. Use of the compound of Formula (II), as defined in claims 1-5, **characterized in that** it is in the manufacture of a medicament used to produce an anti-cancer effect in warm-blooded animals, such as humans.

15. Use of the compound, as define din claims 1-5, **characterized in that** it is in the manufacture of a medicament used in the treatment of diseases related to non-solid tumors such as leukemia, multiple myeloma, hematological malignancies or lymphoma, and to solid tumors and their metastases such as melanoma, non-small cell lung cancer, glioma, hepatocellular carcinoma, glioblastoma, thyroid carcinoma, bile duct, bone, gastric, cerebral/CNS, head and neck, hepatic, stomach, prostatic, breast, renal, testicular, ovarian, skin, cervical, pulmonary, uterine, vulvar, endometrial, colorectal, pancreatic, pleural/peritoneal, membrane, salivary gland and epidermoid tumors.

16. Use of the compound, as defined in claims 1-5, **characterized in that** it is in the manufacture of a medicament for use in carcinoma treatment, prevention or formation inhibition.

17. A method for the treatment of cancer **characterized in that** it employs the compound, as defined in claims 1-5, as single ingredient or in combination with another different anti-cancer agent.
